Europäisches Patentamt

European Patent Office

Office européen des brevets

(11) Publication number: **0 256 735**
A2

# EUROPEAN PATENT APPLICATION

(21) Application number: 87306829.0

(22) Date of filing: 31.07.87

(51) Int. Cl.4: **A61K 37/02** , A61K 39/395 , C07K 7/10

The applicant has filed a statement in accordance with Rule 28 (4) EPC (issue of a sample only to an expert). Accession number(s) of the deposit(s): SSY-1 NCACC 85061901.

(30) Priority: 31.07.86 JP 181014/86

(43) Date of publication of application:
24.02.88 Bulletin 88/08

(84) Designated Contracting States:
DE FR GB

(71) Applicant: KYOWA HAKKO KOGYO CO., LTD.
6-1, Ohte-Machi Itchome
Chiyoda-ku Tokyo(JP)

(72) Inventor: Yokoo, Yoshiharu,c/o Patent Department
Kyowa Hakko Kogyo Co. Ltd 6-1 Ohtemachi Chiyoda-ku Tokyo(JP)
Inventor: Yamasaki, Motoo,c/o Patent Department
Kyowa Hakko Kogyo Co. Ltd 6-1 Ohtemachi Chiyoda-ku Tokyo(JP)
Inventor: Yamaguchi, Kazuo,c/o Patent Department
Kyowa Hakko Kogyo Co. Ltd 6-1 Ohtemachi Chiyoda-ku Tokyo(JP)
Inventor: Hosoi, Shinji c/o Patent Department
Kyowa Hakko Kogyo Co. Ltd 6-1 Ohtemachi Chiyoda-ku Tokyo(JP)

(74) Representative: Lambert, Hugh Richmond et al
D. YOUNG & CO. 10 Staple Inn
London, WC1V 7RD(GB)

(54) T cell-activating factor.

(57) A novel T cell-activating factor is disclosed having a histone H2B-like structure within the molecule and a molecular weight of about 17,500.

EP 0 256 735 A2

## T CELL-ACTIVATING FACTOR

The present invention relates to a novel T cell-activating factor which stimulates the function of immunologically competent cells, and which can potentially be used in the treatment of cancer and other diseases.

Two known T cell-activating factors are so far known, viz. interleukin (IL-1) (Mizel, S.B. et al., J. Immunol., 120, 1497-1503, 1978) and interleukin 2 (IL-2) (Gillis, S. et al., J. Immunol., 120, 2027-2032, 1978).

Human IL-2 has been produced by a recombinant DNA technique using a complementary DNA derived from human IL-2-producing Jurkat cells, and its receptor analysis has also been reported (Taniguchi, T. et al., Nature, 302, 305, 1983; Leonard, W.J. et al., Nature 311, 626-635, 1984).

Also in the case if IL-1, the complementary DNA has recently been analyzed (Auron, D.E. et al., Proc. Natl. Acad. Sci., 81, 7907-7911, 1984; March, C.J., Nature, 315, 641-647, 1985), and the entire amino acid structures of IL-1α (isoelectric point: 5.0) and IL-1β (isoelectric point: 7.0) have been clarified. A variety of IL-1-producing cells is available and includes macrophages, epithelial cells, neutrophiles, B-lymphocytes, etc. (Brunt, J.V., Bio/Technology 3, 595-597, 1985).

In accordance with the present invention a novel T cell-activating factor has been isolated from the supernatant of IL-1 producing cell cultures, and which has different properties from those so far reported for IL-1 and IL-2.

The T cell-activating factor of the present invention has the following physiochemical properties:

a) Molecular weight:

about 17,500 (by SDS-PAGE method) about 20,000 (by gel filtration)

b) Isoelectric point:

9.5 ± 0.3 (by chromatofocusing method) about 9.2 (by isoelectric focusing)

c) Amino acid composition, molar ratio relative to alanine (Ala) (determined by an amino acid analyzer after hydrolysis with 6N hydrochloric acid at 110°C under reduced pressure for 20 hours):

Alanine (Ala) 1.00 Arginine (Arg) 0.60 Aspartic acid/Asparagine (Asp/Asn) 0.38 Cysteine (Cys) not determined Glutamic acid/Glutamine (Glu/Gln) 0.75 Glycine (Gly) 0.59 Histidine (His) 0.21

Isoleucine (Ile) 0.42

Leucine (Leu) 0.46 Lysine (Lys) 1.53 Methionine (Met) 0.24 Phenylalanine (Phe) 0.16 Proline (Pro) 0.46 Serine (Ser) 0.97 Tryptophan (Trp) not determined Threonine (Thr) 0.56 Tyrosine (Tyr) 0.29 Valine (Val) 0.63

d) At the N terminal a 42 amino acid sequence as follows:

$H_2N$-Pro-Glu-Pro-Ala-Lys-Ser-Ala-Pro-Ala-Pro-Lys-Lys-Gly-Ser-Lys-Lys-Ala-Val-Thr-Lys-Ala-Gln-Lys-Lys-Asp-Gly-Lys-Lys-Arg-Lys-Arg-Ser-Arg-Lys-Glu-Ser-Tyr-Ser-Val-Tyr-Val-Tyr-

The novel factor is obtained by culturing human cells capable of producing the factor, e.g. human B lymphoma cell line SSY and Burkitt lymphoma Namalwa cell line, in a culture medium, thereby to accumulate the factor in the culture broth, and recovering it therefrom.

As the culture medium, a protein-free medium such as Ham F10 medium, Ham F12 medium (both are products of Flow lab.), Dulbecco MEM medium, MEM medium, RPMI-1640 medium (the foregoing three are products of Nissui Seiyaku), etc. can be used. If necessary, 1 to 10% (w/v) calf fetal serum, 0.5 to 5 mM/ml glutamine, appropriate amounts of antibiotics [penicillin (25 U/ml), streptomycin (25 μg/ml), etc.], sodium bicarbonate (0.01%), etc. may be added.

Various culture apparatus may be used to perform the culture, e.g. bottles, dishes, roller bottles, spinner flasks, jar fermentors, etc., and culturing is usually carried out at a seed cell density of $5 \times 10^4$ to $2 \times 10^6$ cells/ml, and 30 to 40°C for 2 to 4 days. The amount of factor produced depends upon the cell density, and is formed mainly in the culture liquor. For example, culturing at a seed cell density of $1 \times 10^5$ cells/ml and 37°C for 2 days produces 44 units/ml of the active substance in the culture liquor.

The novel factor can be recovered from the culture broth by salting-out, chromatography, electrophoresis, extraction, centrifugation, dialysis, etc., alone or by an appropriate combination thereof.

Specifically, recovery may be carried out in the following manner. The supernatant of the culture liquor is cooled, centrifuged and concentrated through ultrafiltration by hollow fibers under cooling. The concentrate is then dialyzed against a phosphate buffer and the dialyzate is subjected to column chromatography of QAE type of DEAE type anion exchange resin, for example, DEAE-Toyopearl (product of Toyo Soda) and then eluted at 0 to 1.0 M NaCl gradients. Active fractions are collected, concentrated through an ultrafiltration membrane, subjected to column chromatography of anion exchange resin of high-performance liquid chromatography type, for example, Pharmacia FPLC mono Q (product of Pharmacia Fine Chemicals), and eluted at 0 to 1.0 M NaCl gradients to recover active fractions. The active fractions are concentrated by ultrafiltration, and then subjected to reversed phase high-performance liquid chromatography, and gradient elution of 0.1% trifluoroacetic acid up to 0.1% trifluoroacetic acid containing 70% acetonitrile is carried out. The active fractions are eluted into eluate fractions containing about 45% acetonitrile.

Through analysis by SDS-polyacrylamide gel electrophoresis the active fractions are found to be in a single protein.

Analyses were carried out in the following manner:

1) Molecular weight

A. Determination of molecular weight by sodium dodecylsulfate-polyacrylamide gel electrophoresis (SDS-PAGE):

The SDS-PAGE determination is made using polyacrylamide gel at a concentration of 15% according to the method of Laemmli, et al. (Laemmli, U.K., et al., Nature, 227 680, 1970).

B. Determination of molecular weight by gel filtration using sephadex G-100:

Sephadex G-100 (product of Pharmacia Fine Chemicals) is filled in a column (18 x 240 mm, glass column), and the substance is added thereto, using a buffer of 10 mM phosphate buffer-NaCl (PBS)/0.3 M NaCl (pH 7.2) to carry out gel filtration. The molecular weight is calculated from the elution position using a standard curve obtained by a standard molecular weight kit (product of Pharmacia Fine Chemicals).

2) Isoelectric point:

A. Chromatofocusing method :

The determination of the isoelectric point is carried out in a mono P column for FPLC (0.5 x 20 cm, product of Pharmacia Fine Chemicals) equilibrated with 0.025 M triethylamine-hydrochloric acid buffer (pH 11.0) by elution according to a chromatofocusing method using 0.0075 mmol/pH unit/ml Pharmalyte (pH 8-10.5)-hydrochloric acid (pH 8.0) (Siegel, L.M., Biochimica et Biophysica Acta, 112, 346, 1966). The isoelectric point is determined from found values of pH at the elution position.

B. Isoelectric focusing:

The isoelectric point is determined by an isoelectric point-measuring apparatus) product of Pharmacia Fine Chemicals, Sweden) and an Ampholine polyacrylamide plate (pH 3.5-11.0) prepared according to LKB manual, using a standard isoelectric point measurement market kit (product of Pharmacia Fine Chenicals, Sweden). The isoelectric point is calculated on the basis of the isoelectric point marker after staining by the silver staining method (available from Dai-ichi Kagaku Yakuhin).

(3) Amino acid composition ratio :

Amino acid composition is analyzed by an amino acid analyzer (Pico•Tag, product of Waters Co., USA) after hydrolysis with 6N hydrochloric acid at 110°C (under reduced pressure) for 20 hours.

(4) Amino acid sequence:

The amino acid sequence at the N terminal is determined by combination of an automatic amino acid sequence-measuring apparatus, "Gas phase Protein Sequencer, Model 470A" (product of Applied Biosystem Co.) and a high-performance liquid chromatography (product of Spectra Physics Co.).

(5) Determination of activity:

Activity is evaluated by the method of Kasahara et al. [Rinsho Kensa (J. of Medical Technology), 28 (3), 328, 1984]. That is, thymocytes of 4-to 6-week-old C3H/He mice are suspended in an RPMI-1640 medium (product of Nissui Seiyaku) containing 10% fetal calf serum (FCS) (product of Gibco Co.) and 0.5-1.0 $\mu$g/m$\ell$ concanavalin A (ConA) at a concentration of 1-2x10$^7$ cells/m$\ell$. Then, 0.1 m$\ell$ of the thymocyte suspension and 0.1 m$\ell$ each of samples of the present invention, diluted to appropriate concentrations are distributed into each well of a 96-well microplate and incubated at 37°C in 5% carbon dioxide-containing air for 48 hours. The respective wells are pulse-labeled with 0.25 $\mu$Ci of $^3$H-thymidine, and the cells are recovered on the third day. Incorporated $^3$H-thymidine is then determined by liquid scintillation counting. The activity is expressed on the basis of a capacity of thymocytes to incorporate maximum 50% $^3$H-thymidine as one unit by multiplying it with a dilution ratio of the sample.

Specific activity is expressed on the basis of the amount of protein quantitatively determined by Bradford method (Bradford, N.M., et al., Anal. Biochem., 72, 248, 1976) or from silver staining of SDS-polyacrylamide gel by dividing the activity units calculated in the foregoing method by the amount of protein.

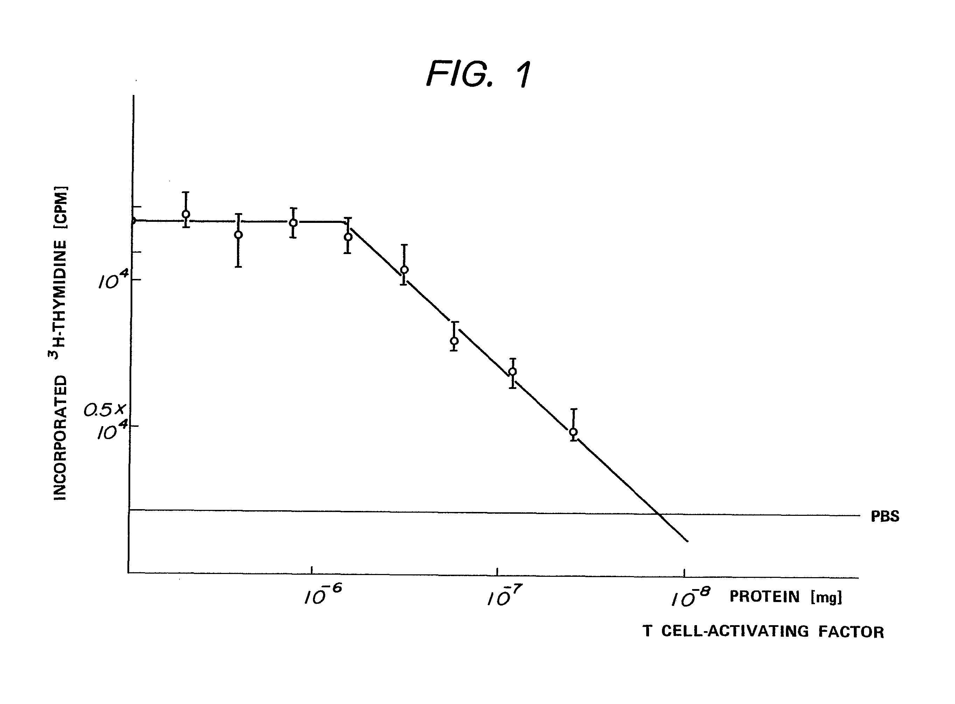

The activity of the T cell-activating factor of this invention obtained using 10$^7$ cells/ml thymocites of C3H/He mice incubated in the presence of 0.5 $\mu$g/ml concanavalin A (ConA) for 48 hours, and incorporation of $^3$H-thymidine for 24 hours is shown in the accompanying drawing.

For comparison human leukemia cell ine HSB.2 (Kasahara, T. et al., J. Immunol., 134, 1682, 1986) was cultured at a concentration of 10$^6$ cells/ml in the presence of 50 $\mu$g/ml phytohemagglutinin (PHA) together with 10 ng/ml of the present factor for 24 hours, and the activity of IL-2 in the supernatant of the culture liquor was determined. The activity of IL-2 was determined as an amount of $^3$H-thymidine incorporated by IL-2-dependent cytotoxic T cell line. The results are shown in Table 1.

Table 1

| T cell-activating factor | IL-2 as formed (cpm) |
|:---:|:---:|
| - | 2,898 $\pm$ 800 |
| + | 15,100 $\pm$ 1,073 |

4

The amino acid sequence of the present factor at the N terminal has the same structure as that of human histone H2B up to 42 amino acids from the N terminal. However, the present factor is different from the human histone H2B in the following points: (1) the present substance has a molecular weight which is about 1,100 larger than that of histone H2B fractionated from human spleen (Ohe, Y. et al., J. Biochem., 85, 615, 1979), and (2) its isoelectric point as a pI value is lower than that of histone H2B by about 1.5.

Also, the present substance has been compared with histone H2B in retention time in reversed phase high-performance liquid chromatography, because the retention time in such a technique depends on the structure and properties of the substance. As a result, it has been found that there is a difference of more than 10 minutes in the retention time. Comparison of the activity shows that histone H2B also has an ability to activate T cells which is about one-tenth of that of the present factor. Thus, it has been found that the present factor differs from histone H2B. A further large difference is that the present factor is a secreted protein found in a supernatant of the medium, whereas histone H2B is a structural protein existing in the nucleus. Thus, it can be seen from the foregoing that the present factor is a novel, physiologically active protein having an ability to activate T cells, a histone H2B-like structure within the molecule and a molecular weight of about 17,500.

The detailed preparation of the factor of this invention is described in the following Example.

## EXAMPLE

### 1) Cell culturing:

Human lymphoma cell line SSY-1 (deposited with the European Collection of Animal Cell Cultures, Great Britain, formerly the National Collection of Animal Cell Cultures, on 19th June, 1985 as NCACC Deposit No. 85061901) was inoculated at a concentration of $5 \times 10^5$ cells/ml in a spinner flask (product of Shibata Seisakusho) containing 800 ml of RPMI-1640 medium (product of Nissui Seiyaku) containing 5% fetal calf serum, and cultured at 37°C for 48 Hours. When the cell concentration reached $2 \times 10^6$ cells/ml, the medium was exchanged with 800 ml of RPMI-1640 medium containing 1% fetal calf serum, and then 100 $\mu$g/ml of silica (product of Mallinckrodt Chemical Works) was added to the medium. The culture meidum was incubated at 37°C for 48 hours, whereby a T cell-activitating factor was accumulated at a concentration of 44.4 units/ml medium. By repeating the same operation as above, 27 1 of a supernatant of culture broth containing the factor was obtained.

### (2) Separation and purification of T cell-activating factor:

#### Purification step 1:

At first, 27 $\ell$ of the supernatant of culture broth was centrifuged in a cooled centrifuge at 4°C and 8,000 rpm for 20 to 30 minutes, and a resulting supernatant solution was about 15-fold concentrated through ultrafiltration hollow fibers (fractionated molecular weight: 3,000, product of Amicon Co.). The concentrated sample was dialyzed up to an NaCl concentration of about 15 mM through the same hollow fibers against 10 mM phosphate buffer (pH 7.5) containing 0.01% polyethyleneglycol 6,000.

#### Purification step 2:

About 2 $\ell$ of the concentrated sample obtained in step 1 was subjected to DEAE-Toyopearl chromatography using a column of DEAE-Toyopearl 650 M (2.8 cm x 40 cm, product of Toyo Soda) equilibrated with 10 mM phosphate buffer (pH 7.5) containing 0.01% polyethyleneglycol 6,000. After filling the sample, the column was washed with the equilibrating buffer, and then eluted at sodium chloride gradients of 0-1.0 M. The active fractions were identified according to the above-mentioned method for determining the activity. The main active fractions were eluted with about 0.6-about 0.8 M NaCl (about 180 m$\ell$). Recovery of the physiologically active fractions in this step was about 23%, and the ratio of purification was about 2,500-fold.

## Purification step 3:

The active fractions obtained in step 2 were collected (180 mℓ) and about 100-fold concentrated (about 2 mℓ) by an ultrafiltration membrane YM5 (product of Amicon Co.). The concentrated sample was diluted with 10 mM tris hydrochloride buffer (pH 7.5) to an NaCl concentration of about 30 mM, and then subjected to adsorption in Pharmacia FPLC mono Q column (5 mm x 50 mm) (product of Pharmacia Fine Chemicals). Then, the column was washed with 10 mM tris hydrochloride buffer (pH 7.5) and eluted at sodium chloride gradients of 0-1 M. Active fractions were eluted with about 0.3-about 0.5 M NaCl (about 15 mℓ). Recovery of activity was about 60% in this step, and the ratio of purification increased to about 1.3-fold.

## Purification step 4:

The active fractions obtained in step 3 were collected (15 mℓ) and about 10-fold concentrated by an ultrafiltration membrane YM5 (product of Amicon Co.). Then, the concentrated sample was subjected to reversed phase high-performance liquid chromatography column YMC-Pack AM-312 ODS (15 cm x 6 mm, product of Kurita Kogyo). The eluate was monitored at an absorbance of 210 nm by Trirotar SR high-performance liquid chromatography (product of Nihon Bunko). Elution was carried out at gradients of 0.1% trifluoroacetic acid to 0.1% trifluoroacetic acid containing 70% acetonitrile at 30°C and a flow rate of 1.0 mℓ/min. The factor was eluted at an acetonitrile concentration of about 42%. The active fraction was analyzed by 15% SDS-polyacrylamide gel electrophoresis, and found to contain a single protein having a molecular weight of about 17,500. Recovery of activity was about 31% in this step, and the ratio of purification was about 25-fold.

Recovery of activity throughout all the steps was about 4%, and the ratio of purification was about $8.5 \times 10^4$-fold. The biological activity of the factor was about $1 \times 10^7$ units/mg protein.

The purification results are summarized in Table 2.

Table 2

Purification of T cell-activating factor

| Purification step | Volume (mℓ) | Protein concentration (μg) | Activity (unit) | Activity recovery (%) | Specific activity (unit/mg protein) | Ratio of purification |
|---|---|---|---|---|---|---|
| Supernatant of culture broth | 27000 | $10.3 \times 10^6$ | $1.2 \times 10^6$ | 100 | 117 | x 1 |
| Eluate from DEAE-Toyopearl | 180 | 946 | $2.8 \times 10^5$ | 23 | $3.0 \times 10^5$ | $2.5 \times 10^3$ |
| Eluate from FPLC mono Q | 15 | 417 | $1.6 \times 10^5$ | 13 | $4.0 \times 10^5$ | $3.4 \times 10^3$ |
| Reversed phase HPLC eluate | 4.0 | 5.0 | $5.0 \times 10^4$ | 4 | $1.0 \times 10^7$ | $8.5 \times 10^4$ |

0 256 735

**Claims**

1. In substantially biologically pure form, a T cell-activating factor having the following physico-chemical properties:

a) Molecular weight: about 17,500 (by SDS-PAGE method)

b) Isoelectric point 9.5 ± 0.3 (by chromatofocusing method)

c) Amino acid composition, molar ratio relative to alanine (determined by an amino acid analyzer after hydrolysis with 6N hydrochloric acid at 110°C under reduced pressure for 20 hours):

Alanine (Ala) 1.00
Arginine (Arg) 0.60
Aspartic acid/Asparagine (Asp/Asn) 0.38
Glutamic acid/Glutamine (Glu/Gln) 0.75
Glycine (Gly) 0.59
Histidine (His) 0.21
Isoleucine (Ile) 0.42
Leucine (Leu) 0.46
Lysine (Lys) 1.53
Methionine (Met) 0.24
Phenylalanine (Phe) 0.16
Proline (Pro) 0.46
Serine (Ser) 0.97
Threonine (Thr) 0.56
Tyrosine (Tyr) 0.29
Valine (Val) 0.63

d) At the N terminal a 42 amino acid sequence as follows:

$H_2N$-Pro-Glu-Pro-Ala-Lys-Ser-Ala-Pro-Ala-Pro-Lys-Lys-Gly-Ser-Lys-Lys-Ala-Val-Thr-Lys-Ala-Gln-Lys-Lys-Asp-Gly-Lys-Lys-Arg-Lys-Arg-Ser-Arg-Lys-Glu-Ser-Tyr-Ser-Val-Tyr-Val-Tyr-.

## FIG. 1

*Axis labels:* INCORPORATED $^3$H-THYMIDINE [CPM] (vertical); $10^4$, $0.5\times 10^4$; PBS; $10^{-6}$, $10^{-7}$, $10^{-8}$ PROTEIN [mg]; T CELL-ACTIVATING FACTOR